# EUROPEAN PATENT APPLICATION

(11) **EP 3 563 843 A1**
(43) Date of publication of application: **06.11.2019**
(21) Application number: 17886713.1
(22) Date of filing: 28.12.2017
(51) Int. Cl.: A61K 31/337, A61K 9/08, A61K 47/10, A61K 47/12, A61K 47/20, A61K 47/22, A61K 47/34, A61P 25/28

(54) **TOPICAL COMPOSITION**

(30) Priority: 28.12.2016 JP 2016255622
(71) Applicant: FUJIFILM Toyama Chemical Co., Ltd., Chuo-ku Tokyo 1040031 (JP)
(72) Inventor: YAMAZAKI Naomi, Ashigarakami-gun Kanagawa 258-8577 (JP); NORO Masaki, Ashigarakami-gun Kanagawa 258-8577 (JP); TSUJIHATA Shigetomo, Ashigarakami-gun Kanagawa 258-8577 (JP)
(74) Representative: Hoffmann Eitle
(86) International application number: PCT/JP2017/047251
(87) International publication number: WO 2018/124281

(57) **Abstract**

An object of the present invention is to provide a composition for external use having an improved skin permeability of 1-(3-(2-(1-benzothiophen-5-yl)ethoxy)propyl)azetidin-3-ol or a salt thereof. The present invention provides a composition for external use comprising 1-(3-(2-(1-benzothiophen-5-yl)ethoxy)propyl)azetidin-3-ol or a salt thereof, one or more solvents selected from the group consisting of alcohols, sulfoxides and amides, and a permeation enhancer.

## Description

### Technical Field

The present invention relates to a composition for external use containing a therapeutic agent for diseases such as Alzheimer's disease.

### Background Art

1-(3-(2-(1-benzothiophen-5-yl)ethoxy)propyl)azetidin-3-ol (hereinafter also referred to as compound A) or a salt thereof has a neuroprotective action, a nerve regeneration promoting action, and a neurite outgrowth action, and is a compound useful as a therapeutic agent for diseases of central and peripheral nerves (Patent Document 1).

For administration routes of compound A or a salt thereof, oral administration is mainly studied. Although there are a few reports on dosage forms other than tablets, Patent Document 2 describes, as a liquid composition for ophthalmic application, a pH 7.5 phosphate buffer solution containing 1 mass/volume% of compound A.

### Prior Art Documents

### Patent Documents

Patent Document 1: International Publication No. WO 2003/035647
Patent Document 2: International Publication No. WO 2004/091605

### Summary of Invention

### Object to be Solved by the Invention

Alzheimer's disease is one kind of dementia that causes reduction of cognitive function as a major symptom. Compound A is one effective therapeutic agent, but it is not easy for patients with Alzheimer's disease to continue to take medicine every day by themselves properly. Thus, caregivers often assist medication. However, such assistance places large burdens on caregivers; and there is a demand to develop a composition for external use such as transdermal preparations including an adhesive preparation and transnasal agents, which enable easy medication and can be expected to reduce caregivers' burdens. In addition, transdermal preparations have an advantage whereby caregivers enable unfailing administration and confirmation of states of administration. Further, an adhesive preparation with a smaller area causes a less burden on a patient, which is expected to further improve medication adherence; and therefore, there is a need to develop a transdermal preparation having a sufficiently high skin permeability. Patent Document 2 discloses in the production example a liquid composition for an eye drop for prevention and/or treatment of retinal nerve diseases, but this has a difficulty in administering a dosage of compound A or a salt thereof required to treat Alzheimer's disease; and it fails to describe the liquid composition used as a transdermal preparation generally capable of administering a larger dosage than an eye drop. In addition, even when the eye drop described in Patent Document 2 is directly applied to the skin, the skin permeability is not sufficient. Further, an increase in the amount of compound A or a salt thereof blended in a preparation disadvantageously reduces the solution stability.

An object to be solved by the present invention is to provide a composition for external use having an improved skin permeability of compound A or a salt thereof.

### Means for Solving the Object

The present inventors have intensively studies to solve the above object, and have found that a combination of compound A or a salt thereof with an appropriate solvent and an appropriate permeation enhancer can significantly improve the skin permeability of compound A or a salt thereof, and have completed the present invention.

That is, the present invention provides the following.
(1) A composition for external use comprising 1-(3-(2-(1-benzothiophen-5-yl)ethoxy)propyl)azetidin-3-ol or a salt thereof, one or more solvents selected from the group consisting of alcohols, sulfoxides and amides, and a permeation enhancer.
(2) The composition for external use according to (1), wherein the solvent is selected from the group consisting of ethanol, benzyl alcohol, propylene glycol, butylene glycol, dipropylene glycol, polyethylene glycol, dimethyl sulfoxide and N-methyl-2-pyrrolidone.
(3) The composition for external use according to (1) or (2), further comprising water.
(4) The composition for external use according to any one of (1) to (3), wherein the permeation enhancer is a compound having a carboxyl group, a hydroxyl group or an alkoxycarbonyl group.
(5) The composition for external use according to any one of (1) to (4), wherein the permeation enhancer is a compound having an aliphatic group having 5 to 18 carbon atoms.
(6) The composition for external use according to any one of (1) to (5), further comprising a water-soluble polymer.
(7) The composition for external use according to (6), wherein the water-soluble polymer is selected from the group consisting of carrageenan, propylene glycol alginate, agar, gelatin, hydroxypropyl cellulose, polyvinyl pyrrolidone, carboxyvinyl polymer, sodium polyacrylate and polyvinyl alcohol.
(8) The composition for external use according to (6), wherein the water-soluble polymer is selected from the group consisting of propylene glycol alginate, agar, gelatin, hydroxypropyl cellulose and polyvinyl pyrrolidone.
(9) The composition for external use according to any one of (1) to (5), further comprising an adhesive agent.
(10) The composition for external use according to any one of (1) to (9), wherein the composition is a transdermal composition for external use.
(11) An adhesive preparation comprising the composition for external use according to any one of (1) to (10).
(12) The adhesive preparation according to (11), wherein the adhesive preparation is a cataplasm or a patch.
(13) The composition for external use according to any one of (1) to (8), wherein the composition is a transnasal composition for external use.

### Advantageous Effects of Invention

The present invention provides a composition for external use having an improved skin permeability of compound A or a salt thereof.

### Embodiment of Carrying out the Invention

In the present invention, the numerical range expressed by "to" includes values at both ends unless otherwise described.

The composition for external use of the present invention contains compound A or a salt thereof, one or more solvents selected from the group consisting of alcohols, sulfoxides and amides, and a permeation enhancer. In the present invention, a composition for external use having a significantly increased skin permeability is obtained by blending a specific solvent and permeation enhancer with compound A or a salt thereof. It should be noted that even application to the skin of pH 7.5 phosphate buffer solution containing 1 mass/volume% of compound A described in Patent Document 2 provides a low skin permeability, so it was difficult to administer a dosage required to exhibit drug efficacy.

### <Compound A or salt thereof>

In the present invention, compound A or a salt thereof is used as an active ingredient.

Since compound A has a cyclic amino group, examples of a salt thereof include salts commonly known in basic groups.

Examples of salts in basic groups include salts with mineral acids such as hydrochloric acid, hydrobromic acid, nitric acid, and sulfuric acid; salts with organic carboxylic acids such as formic acid, acetic acid, citric acid, oxalic acid, fumaric acid, maleic acid, succinic acid, malic acid, tartaric acid, aspartic acid, trichloroacetic acid, and trifluoroacetic acid; and salts with sulfonic acids such as methanesulfonic acid, benzenesulfonic acid, p-toluenesulfonic acid, mesitylene sulfonic acid, and naphthalene sulfonic acid.

Among the above salts, examples of preferable salts include pharmacologically acceptable salts and examples of more preferable salts include salts with maleic acid.

In the case of having isomers (for example, optical isomers, geometric isomers, and tautomers), compound A or a salt thereof may be any of all these isomers and may be any of hydrates, solvates, and all crystal forms.

Compound A or a salt thereof can be manufactured by methods known per se or combinations thereof, or a method described in Patent Document 1.

The content of compound A or a salt thereof in the composition for external use of the present invention is not particularly limited, and it is generally 0.1 to 30 mass%, preferably 0.5 to 25 mass%, more preferably 1 to 20 mass%, and further preferably 2 to 15 mass% based on the total mass of the composition.

### <Solvent>

The solvent used in the present invention is one or more selected from the group consisting of alcohols, sulfoxides and amides.

In the present invention, a solvent that can dissolve compound A or a salt thereof is preferably used. Since it is desired to allow a required amount of compound A or a salt thereof to efficiently permeate into the blood, a solvent that can dissolve compound A or a salt thereof at a high concentration is more preferably used. From the above viewpoint, the present invention uses alcohols, amides or sulfoxides having a relatively higher polarity among solvents. In the present invention, the above solvents can be used in combination of two or more thereof.

Examples of alcohols include: monohydric alcohols such as ethanol, 1-propanol, 2-propanol, 1-butanol, 2-butanol, isobutyl alcohol, tert-butyl alcohol and benzyl alcohol; and polyhydric alcohols such as ethylene glycol, 1,2-propanediol (propylene glycol), 1,3-propanediol, dipropylene glycol, 1,3-butanediol (butylene glycol), 1,4-butanediol, glycerin, polyethylene glycol and polypropylene glycol.

Examples of sulfoxides include dimethyl sulfoxide (DMSO) and tetramethylene sulfoxide.

Examples of amides include N,N-dimethyl acetamide, N-methyl-2-pyrrolidone and N,N'-dimethyl imidazolidinone.

Among these solvents, a solvent having a high solubility of compound A and a salt thereof is preferred. Table 1 shows solubility of a maleate of compound A, which is a representative salt of compound A, in representative solvents. Examples of more preferable solvents include solvents having a solubility of a maleate of compound A of 2 g/100 g solvent or more at 25°C, specifically, ethanol, benzyl alcohol, propylene glycol, butylene glycol, dipropylene glycol, polyethylene glycol, N-methyl-2-pyrrolidone and dimethyl sulfoxide. Among them, a solvent having a solubility of compound A of 5 g/100 g solvent or more is further preferable, and that having the solubility of 10 g/100 g solvent or more is the most preferable.

**[Table 1]**

| | Solubility of maleate of compound A at 25°C (g/100 g of solvent) |
|---|---|
| Ethanol | 3 |
| Benzyl alcohol | 35 |
| Propylene glycol | 10 |
| Butylene glycol | 5 |
| Dipropylene glycol | 8 |
| Polyethylene glycol 200 | 10 |
| Polyethylene glycol 400 | 16 |
| N-methyl-2-pyrrolidone | >50 |
| Dimethyl sulfoxide | >50 |

| | |
|---|---|
| Polyethylene glycol (number average molecular weight 200) Polyethylene glycol (number average molecular weight 400) | |

Further, among the above-described solvents, solvents having an effect of suppressing the decomposition of compound A or a salt thereof are preferable. From the viewpoint, specific examples of preferable solvents include polyhydric alcohols such as ethylene glycol, 1, 2-propanediol (propylene glycol), 1,3-propanediol, dipropylene glycol, 1,3-butanediol (butylene glycol), 1,4-butanediol, glycerin, polyethylene glycol and polypropylene glycol; sulfoxides such as dimethyl sulfoxide (DMSO) and tetramethylene sulfoxide; and more preferable examples include 1,2-propanediol (propylene glycol) and dimethyl sulfoxide (DMSO).

The content of a solvent in the composition for external use of the present invention is not particularly limited. A lower limit of the content of the solvent is generally 20 mass% or more, and preferably 30 mass% or more based on the total mass of the composition. The lower limit of the content of the solvent may be 35 mass% or more, 40 mass% or more, 45 mass% or more, 50 mass% or more, 55 mass% or more, 60 mass% or more, 65 mass% or more, and 70 mass% or more. An upper limit of the content of the solvent is generally 99 mass% or less, preferably 98 mass% or less, and more preferably 97 mass% or less based on the total mass of the composition. The upper limit of the content of the solvent may be 95 mass% or less, 90 mass% or less, 85 mass% or less and 80 mass% or less.

### <Permeation enhancer>

The composition for external use of present invention contains a permeation enhancer. The permeation enhancer is a substance that reduces barrier function of a horny layer against a drug and improves the skin permeability.

Examples of major permeation enhancers include:
substances which interact with horny layer lipid to enhance the structural change of lipidic membranes or the fluidity, thereby increasing a diffusion coefficient of a drug in a horny layer (AZONE (1-dodecyl-azacycloheptan-2-one), terpenes, fatty acids, fatty acid esters, surfactants, alcohols and the like);
substances which interact with keratin protein in horny layer cells to loosen the high-density structure of protein, thereby increasing a diffusion coefficient of a drug in a horny layer (dimethyl sulfoxide, ionic surfactants and the like); and
solvents which permeate a horny layer to improve the chemical environment or the solubility in the horny layer, thereby enhancing the distribution of a drug to the horny layer (water, ethanol, propylene glycol, or the like),.

It is generally known that an organic solvent has an effect as an permeation enhancer; however, in the present invention, , a substance that can further enhance the skin permeability by addition of itself in addition to one or more solvents selected from the group consisting of the above-described alcohols, sulfoxides and amides is referred to as a permeation enhancer.

The permeation enhancer is not particularly limited as long as it is applicable to pharmaceuticals, so alcohols, carboxylic acids, esters, ethers or the like can be used.

Specific examples of the permeation enhancer include: alcohols such as hexanol, octanol, decanol, lauryl alcohol, myristyl alcohol, cetyl alcohol, stearyl alcohol, isostearyl alcohol, oleyl alcohol, behenyl alcohol, octyl dodecanol, cetostearyl alcohol, hexyl decanol, polyoxyethylene capryl ether, polyoxyethylene octyl ether, polyoxyethylene decanyl ether, polyoxyethylene lauryl ether, polyoxyethylene myristyl ether, polyoxyethylene cetyl ether, polyoxyethylene isocetyl ether, polyoxyethylene cetostearyl ether, polyoxyethylene stearyl ether, polyoxyethylene isostearyl ether, polyoxyethylene oleyl ether, polyoxyethylene behenyl ether, polyoxyethylene cholesteryl ether, polyoxyethylene tridecyl ether, polyoxyethylene methyl glucoside, polyethylene glycol caprate, polyethylene glycol laurate, polyethylene glycol myristate, polyethylene glycol palmitate, polyethylene glycol stearate, polyethylene glycol isostearate, polyethylene glycol oleate, polyethylene glycol distearate, polyoxyethylene polyoxypropylene glycol, polyoxyethylene hydrogenated castor oil, polyoxyethylene castor oil, polyoxyethylene sorbitan monolaurate, polyoxyethylene sorbitan monostearate, polyoxyethylene sorbitan tristearate, polyoxyethylene sorbitan monooleate, and polyoxyethylene sorbitan monopalmitate; carboxylic acids such as caproic acid, caprylic acid, capric acid, lauric acid, myristic acid, palmitic acid, stearic acid, oleic acid, sorbic acid, levulinic acid, isostearic acid, and behenic acid; esters such as sorbitan monocaprate, sorbitan monolaurate, sorbitan monomyristate, sorbitan monopalmitate, sorbitan isostearate, sorbitan monooleate, sorbitan monostearate, sorbitan sesquioleate, sorbitan tristearate, glycerol monooleate, ethylhexyl salicylate, isopropyl myristate, diethyl sebacate, cetyl palmitate, diisopropyl adipate, diisobutyl adipate, diisopropyl dilinoleate, ethyl oleate, isostearyl isostearate, isopropyl palmitate, isopropyl stearate, methyl laurate, methyl stearate, oleyl oleate, myristyl lactate, and propylene glycol diacetate; ethers such as dimethyl isosorbide; and sodium cocoyl sarcosinate, sodium laureth sulfate, dipotassium glycyrrhizinate, urea, cyclomethicone, gluconolactone, lemon oil, menthol, limonene, and α-terpinenol.
permeation enhancers may be used singly or in a combination of two or more.

Since a compound having a carboxyl group, a hydroxyl group or an alkoxycarbonyl group has a profound effect as an permeation enhancer, it is preferable as the permeation enhancer of the present invention.

Examples of the compound having a carboxyl group, a hydroxyl group or an alkoxycarbonyl group include carboxylic acids, alcohols and esters.

In addition, it is preferred to use a permeation enhancer having an aliphatic group having 5 to 18 carbon atoms as a hydrophobic group, and more preferred to use a permeation enhancer having an aliphatic group having 9 to 18 carbon atoms. Examples of the aliphatic group include a saturated alkyl group, an unsaturated alkyl group, a saturated alkylene group, and an unsaturated alkylene group, and each of them may have a branched structure or a ring structure, and may have a substituent. Examples thereof include a hexyl group, an octyl group, a 2-ethylhexyl group, a decyl group, a lauryl group, a myristyl group, a palmityl group, a stearyl group, an isostearyl group, and an oleyl group.

More preferred is a permeation enhancer having a carboxyl group or a hydroxyl group as a hydrophilic group, and an aliphatic group having 5 to 18 carbon atoms as a hydrophobic group; and the most preferred is a permeation enhancer having a carboxyl group or a hydroxyl group as a hydrophilic group and an aliphatic group having 9 to 18 carbon atoms as a hydrophobic group. Specifically, more preferred are caproic acid, caprylic acid, capric acid, lauric acid, myristic acid, palmitic acid, stearic acid, isostearic acid, oleic acid, hexanol, octanol, decanol, lauryl alcohol, myristyl alcohol, cetyl alcohol, stearyl alcohol, isostearyl alcohol, oleyl alcohol, polyoxyethylene capryl ether, polyoxyethylene octyl ether, polyoxyethylene decanyl ether, polyoxyethylene lauryl ether, polyoxyethylene myristyl ether, polyoxyethylene cetyl ether, polyoxyethylene isocetyl ether, polyoxyethylene cetostearyl ether, polyoxyethylene stearyl ether, polyoxyethylene isostearyl ether, and polyoxyethylene oleyl ether; and the most preferred are capric acid, lauric acid, myristic acid, palmitic acid, stearic acid, isostearic acid, oleic acid, decanol, lauryl alcohol, myristyl alcohol, cetyl alcohol, stearyl alcohol, isostearyl alcohol, oleyl alcohol, polyoxyethylene decanyl ether, polyoxyethylene lauryl ether, polyoxyethylene myristyl ether, polyoxyethylene cetyl ether, polyoxyethylene isocetyl ether, polyoxyethylene cetostearyl ether, polyoxyethylene stearyl ether, polyoxyethylene isostearyl ether, and polyoxyethylene oleyl ether.

The content of the permeation enhancer in the composition for external use of the present invention is not particularly limited, and it is generally 0.1 to 30 mass%, preferably 0.2 to 25 mass%, and more preferably 0.5 to 20 mass% based on the total mass of the composition.

### <Water>

The composition for external use of the present invention may further contain water.

In the case that the composition of the present invention contains water, purified water, distilled water, deionized water, pure water, ultrapure water, water for injection or the like is preferred as water, from the viewpoint of good biocompatibility and a smaller amount of impurities.

In the case that the composition of the present invention contains water, by containing water, increase of a skin permeation ratio of compound A or a salt thereof in the composition of the present invention, and improvement of suitability for formulation by enhancement of the blendability with a water-soluble additive (gelling agent, thickener or the like) are observed. Meanwhile, containing of water tends to lower the solubility of a permeation enhancer. Thus, the water content is preferably in such a range that can maintain a solubility state of each component contained in the composition of the present invention. From the above viewpoint, in the case that the composition of the present invention contains water, a preferable water content is preferably in the range of 1 mass% to 80 mass%, more preferably in the range of 2 mass% to 70 mass%, further preferably in the range of 5 mass% to 50 mass%, and the most preferably in the range of 5 mass% to 35 mass% based on the total mass of the composition. In the case that the composition for external use of the present invention contains water as a solvent, as long as the water content is within the above ranges, the skin permeation efficiency of compound A or a salt thereof is enhanced and also the suitability for formulation is more excellent.

### <Water-soluble polymer>

When the composition for external use of the present invention is used as an adhesive preparation like a cataplasm, or a gel, the composition for external use of the present invention can further contain a water-soluble polymer.

As the water-soluble polymer, for example, the following polymers can be used, but the water-soluble polymer is not limited thereto.

Plant-derived naturally-occurring polymers such as guar gum, locust bean gum, carrageenan, alginic acid, sodium alginate, propylene glycol alginate, agar, gum arabic, gum tragacanth, karaya gum, pectin or starch;
Microorganism-derived naturally-occurring polymers such as xanthan gum or acacia gum;
Animal-derived naturally-occurring polymers such as gelatin or collagen;
Cellulose-based semi-synthetic polymers such as methyl cellulose, ethyl cellulose, hydroxyethyl cellulose, hydroxypropyl cellulose or sodium carboxymethyl cellulose;
Starch-based semi-synthetic polymers such as soluble starch, carboxymethyl starch or dialdehyde starch;
Synthetic vinyl polymers such as polyvinyl alcohol, polyvinyl pyrrolidone, polyvinyl methacrylate or carboxyvinyl polymer;
Synthetic acrylic polymers such as polyacrylic acids or sodium polyacrylate; and
Synthetic polymers such as polyethyleneoxide or methyl vinyl ether/maleic anhydride copolymers.

Among these, those having a high solubility in a mixture solution of a solvent and water used in the present invention are preferred; and more preferred are, for example, those having a high solubility in a mixture solution having a solvent content as in propylene glycol/water = 7/3, such as carrageenan, propylene glycol alginate, agar, gelatin, hydroxypropyl cellulose, polyvinyl pyrrolidone, carboxyvinyl polymer, sodium polyacrylate or polyvinyl alcohol. Further preferred is propylene glycol alginate, agar, gelatin, hydroxypropyl cellulose or polyvinyl pyrrolidone, which are capable of blending the salt of compound A at a high concentration.

The above-described water-soluble polymers may be used singly or may be used in appropriate combinations of two or more thereof.

In the case that the composition for external use of the present invention contains a water-soluble polymer, the content of the water-soluble polymer in the composition for external use of the present invention is not particularly limited, and it is generally 0.1 to 15 mass%, preferably 0.1 to 12 mass%, and more preferably 0.2 to 10 mass% based on the mass of the composition for external use.

### <Adhesive agent>

When the composition for external use of the present invention is used as an adhesive preparation such as a tape, the composition for external use of the present invention may contain an adhesive agent.

Examples of the adhesive agent include natural rubber, synthetic rubber, silicone and elastomer, such as natural rubber, isoprene rubber, polyisobutylene, a styrene-isoprene-styrene block copolymer, a styrene-butadiene-styrene block copolymer, a styrene-ethylene-butylene-styrene block copolymer, (meth)acrylic acid alkyl ester (co)polymer, polybutene, and liquid polyisoprene.

The content of the adhesive agent in the composition for external use of the present invention is not particularly limited, and it is generally 1 mass% to 80 mass%, preferably 5 mass% to 65 mass%, and more preferably 10 mass% to 50 mass% based on the mass of the composition for external use.

When the composition for external use of the present invention contains an adhesive agent, it may contain a known tackifier with the purpose of controlling the physical properties of the composition for external use. Preferable examples of the tackifier include a petroleum resin (for instance, an aromatic petroleum resin, an aliphatic petroleum resin and a resin from C9 fraction), a terpene resin (for instance, an α pinene resin, a β-pinene resin, a terpene phenol copolymer, a hydrogenated terpene phenol resin, an aromatic modified hydrogenated terpene resin and abietate-based resin), a rosin-based resin (for instance, a partial hydrogenated gum rosin resin, an erythritol modified wood rosin resin, a tall oil rosin resin and a wood rosin resin), a cumarone-indene resin (for instance, a cumarone-indene styrene copolymer), and a styrene-based resin (for instance, polystyrene, and a copolymer of styrene and α-methylstyrene).

In addition, the composition for external use of the present invention may contain a softening agent. Examples of the softening agent include: a petroleum softening agent such as liquid paraffin, processing oil or low-molecular polybutene; a fatty acid-based softening agent such as coconut oil or castor oil; and purified lanoline.

Further, with the purpose of controlling the adhesiveness of a tape or the like, the composition for external use of the present invention may, where necessary, contain a filler such as zinc oxide, titanium oxide, calcium carbonate or silicic acids.

When the tape contains an adhesive agent, it may contain a known tackifier with the purpose of controlling the physical properties of a transdermal preparation.

### <Form of composition for external use>

The dosage form of the composition for external use of the present invention is not particularly limited, and it may be produced in the form of a transdermal composition for external use (transdermal preparation), a transnasal composition for external use (transnasal preparation) or the like.

### [Transdermal composition for external use (transdermal preparation)]

Regarding a transdermal composition for external use (transdermal preparation) of the present invention, the dosage form thereof is not particularly limited as long as it keeps compound A or a salt thereof as the active ingredient on the skin for a desired time period, and it may be produced in the form of an adhesive preparation, an ointment or the like.

### (Adhesive preparation)

Examples of the adhesive preparation include a cataplasm, a patch or a tape. Further, an adhesive preparation has such a form that the composition for external use of the present invention containing compound A or a salt thereof: is applied to an appropriate base material together with an adhesive layer; or is arranged in a drug storage layer between the base material and a release control layer. Alternatively, it may have a form wherein the composition for external use of the present invention is sealed between the base material and the release control layer.

As the cataplasm, generally used is one having a water-containing adhesive agent layer spread on one surface of a stretchable support, wherein a surface of the water-containing adhesive agent layer is covered with a plastic film. The composition for external use of the present invention can be preferably used as a water-containing adhesive agent layer of a cataplasm. When the composition for external use of the present invention is used as a water-containing adhesive agent, it may further contain, for example, a hardening agent, a hardening regulating agent and a moistening agent; it contains moisture so that drug efficacy to the skin can be sufficiently obtained and has adhesiveness; and it is formed so as to be softened even at ordinary temperature or higher and have an appropriate cohesion force in such a degree as to leave no plaster on the skin. When the composition for external use of the present invention is used for a cataplasm, preferable water-soluble polymers are the same as described above; but, in particular, the composition preferably contains at least either one of gelatin and agar having a high gelation capability.

With respect to components other than the water-soluble polymer, those described in, for example, JP Patent Publication (Kokai) No. 10-95728 A (1998) (paragraphs 0018 to 0031) or JP Patent Publication (Kokai) No. 2006-320745 A (paragraphs 0013 to 0016) can be preferably used.

The composition for external use of the present invention can be preferably used as a patch. A patch is generally composed of a support, a drug storage layer containing a drug-containing fluid, a release control film for supplying the drug to a skin surface or mucous surface, a pressure sensitive adhesive layer for adhering a preparation to the skin or mucous surface, a release film for protecting a the pressure sensitive adhesive agent layer, and the like. The composition for external use of the present invention is filled into the drug storage layer formed between the support and the release control film. The pressure sensitive adhesive agent layer may cover the entire surface of the release control film, and it may cover only a peripheral region other than the vicinity to the center of the release control film. For uses of the preparation, the release film is peeled off; the pressure sensitive adhesive agent layer is brought into contact with the skin surface or the mucous surface to fix the preparation and this state is maintained; and thereby, compound A or a salt thereof passes from the drug storage layer through a drug release layer to the skin surface or the mucous surface, transdermally or transmucosally moving into the body. For the composition for external use of the present invention, any device other than the above may be used. Any known material may be used for the support, the release control film, the pressure sensitive adhesive agent, the release film or the like.

When the composition for external use of the present invention is used as a tape, it may contain an adhesive agent.

### (Ointment)

Examples of an ointment include a gel, a cream, a salve and a liquid (a lotion and a liniment).

The composition for external use of the present invention is not particularly limited within the limit of transdermal applicability as long as it can be applied, sprayed or adhered directly to a site (diseased site) in need thereof of the skin. The form as a preparation of the composition for external use of the present invention is a composition for external use for, for example, a lotion, a liniment, a gel, a cream, a salve and a spray.

### <Other component that may be contained in transdermal composition for external use (transdermal preparation) of the present invention>

A transdermal preparation can contain a known additive depending on the purpose in the dosage form of a transdermal preparation in addition to the above-described active ingredient, solvent, permeation enhancer, water, adhesive agent and water-soluble polymer, as long as the effects thereof are not damaged.

Examples of the other components usable for the transdermal preparation include a solvent other than the above-described, a moistening agent, an emollient, a skin barrier agent, a surfactant, a thickener, organic particles, inorganic particles, a buffer, a pH adjuster, a coloring agent, a perfume and a crosslinking agent. In addition, with the purpose of improving the stability of the preparation, a known stabilizer, antioxidant or the like may be contained.

### [Transnasal composition for external use (transnasal preparation)]

The composition for external use of the present invention may be formulated as a transnasal composition for external use (transnasal preparation). The dosage form of the transnasal preparation is not particularly limited as long as it can be transnasally administered or used as a nasal drop; and it may be in the form of a semisolid agent such as a salve, a cream and a gel, and a liquid (a solution agent, an emulsion, a suspension and the like).

The transnasal preparation of the present invention may further contain one or more selected from a vehicle (a transnasal vehicle or a carrier), a moistening agent, a gel or a thickening agent, an isotonizing agent, a pH adjuster, an emulsifier, a surfactant, a buffer, an osmoregulating agent, a preservative, an antiseptic, a refrigerant and a perfume depending on its dosage form.

The present invention will be described further in detail by referring to the following examples, but the present invention is not limited by these examples. The numeral values described in Tables 2 to 10 represent contents (mass%) of respective components based on the total amount of the composition.

### Examples

### <Examples 1 to 34 and Comparative Examples 1 to 11>

In accordance with the compositions shown in Tables 2 to 8, components shown in each table were stirred in a container, and a solution of a maleate of compound A was prepared.

Details on each component described in each table are as follows.
- Propylene glycol (manufactured by JT Baker)
- Ethanol (manufactured by Wako Pure Chemical Industries, Ltd.)
- Dimethyl sulfoxide (manufactured by Wako Pure Chemical Industries, Ltd.)
- N-methyl pyrrolidone (manufactured by Wako Pure Chemical Industries, Ltd.)
- Polyethylene glycol 200 (manufactured by Wako Pure Chemical Industries, Ltd.)
- Polyethylene glycol 400 (manufactured by Wako Pure Chemical Industries, Ltd.)
- Capric acid (manufactured by MP biochemicals)
- Lauric acid (manufactured by Tokyo Chemical Industry Co., Ltd.)
- Stearic acid (manufactured by Wako Pure Chemical Industries, Ltd.)
- Isostearic acid (Isostearic Acid EX manufactured by Kokyu Alcohol Kogyo Co., Ltd.)
- Oleic acid (SR OLEIC ACID-LQ-(JP) manufactured by Croda)
- Hexanol (manufactured by Wako Pure Chemical Industries, Ltd.)
- Octanol (manufactured by Wako Pure Chemical Industries, Ltd.)
- Decanol (manufactured by Tokyo Chemical Industry Co., Ltd.)
- Lauryl alcohol (Conol 20P, manufactured by New Japan Chemical Co., Ltd.)
- Isostearyl alcohol (Isostearyl Alcohol EX manufactured by Kokyu Alcohol Kogyo Co., Ltd.)
- Oleyl alcohol (Tokyo Chemical Industry Co., Ltd.)
- POE (2) lauryl ether (NIKKOL (R) BL-2 manufactured by Nikko Chemicals Co., Ltd.) (POE is an abbreviation for polyoxyethylene)
- POE (2) oleyl ether (NIKKOL (R) BO-2V manufactured by Nikko Chemicals Co., Ltd.)
- POE (7) oleyl ether (NIKKOL (R) BO-7V manufactured by Nikko Chemicals Co., Ltd.)
- Isopropyl palmitate (manufactured by Wako Pure Chemical Industries, Ltd.)
- Isopropyl myristate (manufactured by Wako Pure Chemical Industries, Ltd.)

### <Skin permeability of maleate of compound A)

The skin permeability can be evaluated by an *in vitro* skin permeation experimental method or an *in vivo* skin permeation experimental method. Examples of the *in vitro* skin permeation experimental method include a method using a diffusion cell. Examples of the diffusion cell include a vertical cell such as a Franz diffusion cell, or a horizontal cell. The diffusion cell is composed of two cell parts and is used with a membrane for measuring the permeability clamped between the two cell parts. Examples of the membrane include human skin, animal skin, a three-dimensional culture skin model, or an artificial membrane. In the examples, the evaluation was made by a skin permeability test using the skin isolated from a hairless rat shown below.

### <Skin permeability test using skin isolated from hairless rat>

The skin isolated from an 8-week old SPF (Specific Pathogen Free) hairless rat (Ishikawa Laboratory Animals) is mounted on a Franz diffusion cell (PermeGear, Inc., a jacketed stationary type, effective permeation area: 1 cm², receptor volume: 8 mL) in such a direction that the horny layer of the skin faced up.

A composition to be evaluated is applied uniformly to a horny layer side of a top surface of the skin at 100 mg/cm², and a concentration of the maleate of compound A, which was eluted through the skin into a receptor fluid filled into the cell, is measured. For the diffusion cell, water at 32°C is circulated in a jacket to keep a surface temperature of the skin, and a phosphate buffer solution with a pH of 7.4 is used as the receptor fluid. In a closed state, the receptor fluid is stirred with a magnetic stirrer; after 24 hours, the receptor fluid is collected, and replaced with a new receptor fluid having the same volume as that of the collected one. A concentration of compound A in the collected receptor fluid is measured by use of a high performance liquid chromatography (Prominence UFLCXR: trade name, manufactured by Shimadzu Corporation), and thereby, the amount of compound A permeating the skin is calculated.

### <Evaluation on skin permeation of compound A>

### (Evaluation 1) Amount of compound A permeating skin

The skin permeability was evaluated by obtaining a ratio of the amount of compound A that permeated the skin from the composition of each Example or each Comparative Example based on the amount of compound A that permeated the skin from the composition of Comparative Example 1. A larger amount of compound A permeating the skin is preferred. Evaluation criteria are shown below.
D: Less than 1.2 times relative to Comparative Example 1
C: 1.2 times or more to less than 2 times relative to Comparative Example 1
B: 2 times or more to less than 5 times relative to Comparative Example 1
A: 5 times or more relative to Comparative Example 1

### (Evaluation 2) Increase ratio of amount permeating skin due to permeation enhancer

An increase ratio of an amount permeating the skin in an experimental example with addition of a permeation enhancer to an amount permeating the skin in an experimental example with no permeation enhancer was calculated, and an enhancing effect of the permeation enhancer on the amount permeating the skin was evaluated. As the increase ratio was larger, the effect of the permeation enhancer was shown to be more profound; and the ability of the permeation enhancer is considered high. Evaluation criteria are shown below.
D: Less than 2 times relative to the experimental example with no permeation enhancer
C: 2 times or more to less than 5 times relative to the experimental example with no permeation enhancer
B: 5 times or more to less than 20 times relative to the experimental example with no permeation enhancer
A: 20 times or more relative to the experimental example with no permeation enhancer

### (Evaluation 3) Ratio of compound A that has permeated skin

A ratio of an amount permeating the skin to an amount of compound A in the composition which was used for the skin permeation test was calculated, and thereby, a skin permeation efficiency of compound A contained in each composition was evaluated. A comparison on the skin permeation efficiency between an experimental example containing a permeation enhancer and an experimental example containing no permeation enhancer allows us to find the skin permeation promoting effect of the permeation enhancer. A higher efficiency enables compound A as the active ingredient in the composition to be efficiently absorbed in the body, and it is preferred. Evaluation criteria are shown below.
D: Less than 35%
C: 35% or more to less than 55%
B: 55% or more to less than 75%
A: 75% or more

The above Evaluations 2 and 3 are both criteria for evaluating the effect of the permeation enhancer, so evaluation results on Evaluation 1, 2 or 3 on each experimental example are shown in each table.

**[Table 2]**

| | Comparative Example 1 | Comparative Example 2 |
|---|---|---|
| Maleate of compound A | 1 | 1 |
| Phosphate buffer at pH 7.5 | 99 | |
| Water | | 99 |
| Amount of compound A that permeated skin (µg/cm²) | 320 | 100 |
| Ratio of compound A that permeated skin | 32% | 10% |
| Evaluation 1: Amount of compound A that permeated skin | D | D |
| Evaluation 3: ratio of compound A thatpermeated skin | D | D |

When the fluid of Comparative Example 1 was applied to the skin, a required amount of compound A did not permeate into the skin.

**[Table 3-1]**

| | Comparative Example 3 | Example 1 | Example 2 | Example 3 | Example 4 | Example 5 | Example 6 |
|---|---|---|---|---|---|---|---|
| Maleate of compound A | 10 | 10 | 10 | 10 | 10 | 10 | 10 |
| Propylene glycol | 90 | 89 | 89 | 89 | 89 | 89 | 89 |
| Lauric acid | | 1 | | | | | |
| Isostearic acid | | | 1 | | | | |
| Oleic acid | | | | 1 | | | |
| Decanol | | | | | 1 | | |
| Lauryl alcohol | | | | | | 1 | |
| Isostearyl alcohol | | | | | | | 1 |
| Amount of compound A permeating skin (µg/cm²) | **80** | **1310** | **2360** | **2690** | **4380** | **6380** | **1100** |
| Increase ratio of amount permeating skin by permeation enhancer | 1 | 16 | 30 | 34 | 55 | 80 | 14 |
| Evaluation 1: Amount of compound A that permeated skin | D | B | A | A | A | A | B |
| Evaluation 2: increase ratio of amount permeating skin due to permeation enhancer | - | B | A | A | A | A | B |

**[Table 3-2]**

| | Example 7 | Example 8 | Example 9 |
|---|---|---|---|
| Maleate of compound A | 10 | 10 | 10 |
| Propylene glycol | 89 | 89 | 89 |
| Oleyl alcohol | 1 | | |
| POE(2) lauryl ether | | 1 | |
| POE(2) oleyl ether | | | 1 |
| Evaluation 1: Amount of compound A permeating skin (µg/cm²) | **1770** | **2830** | **2480** |
| Evaluation 2: increase ratio of amount permeating skin due to permeation enhancer | 21.52 | 34.55 | 30.21 |
| Evaluation 1: Amount of compound A that permeated skin | A | A | A |
| Evaluation 2: increase ratio of amount permeating skin due to permeation enhancer | A | A | A |

**[Table 3-3]**

| | Comparative Example 3 | Example 10 | Example 11 | Example 12 |
|---|---|---|---|---|
| Maleate of compound A | 10 | 10 | 10 | 10 |
| Propylene glycol | 90 | 89 | 89 | 89 |
| POE(7) oleyl ether | | 1 | | |
| Isopropyl palmitate | | | 1 | |
| Isopropyl myristate | | | | 1 |
| Amount of compound A permeating skin (µg/cm²) | **80** | **320** | **240** | **220** |
| Increase ratio of amount permeating skin by permeation enhancer | 1 | 4 | 3 | 3 |
| Evaluation 2: increase ratio of amount permeating skin due to permeation enhancer | - | C | C | C |

Addition of a permeation enhancer to a propylene glycol solution having a maleate of compound A dissolved therein greatly increased the amount (of the compound A) that permeated through the skin up to about 80 times.

**[Table 4-1]**

| | Comparative Example 4 | Example 13 | Example 14 | Example 15 | Example 16 |
|---|---|---|---|---|---|
| Maleate of compound A | 2 | 2 | 2 | 2 | 2 |
| Ethanol | 98 | 97 | 97 | 97 | 97 |
| Lauric acid | | 1 | | | |
| Stearic acid | | | 1 | | |
| Isostearic acid | | | | 1 | |
| Oleic acid | | | | | 1 |
| Amount of compound A that permeated skin (µg/cm²) | **450** | **860** | **1050** | **810** | **1020** |
| Ratio of compound A that permeated skin | 23% | 43% | 53% | 41% | 51% |
| Evaluation 1: Amount of compound A that permeated skin | C | B | B | B | B |
| Evaluation 3: ratio of compound A that permeated skin | D | C | C | C | C |

**[Table 4-2]**

| | Example 17 | Example 18 | Example 19 | Example 20 | Example 21 |
|---|---|---|---|---|---|
| Maleate of compound A | 2 | 2 | 2 | 2 | 2 |
| Ethanol | 97 | 97 | 97 | 97 | 97 |
| Decanol | 1 | | | | |
| Lauryl alcohol | | 1 | | | |
| Isostearyl alcohol | | | 1 | | |
| Oleyl alcohol | | | | 1 | |
| POE(2) oleyl ether | | | | | 1 |
| Amount of compound A that permeated skin (µg/cm²) | **1170** | **1270** | **1180** | **1250** | **1320** |
| Ratio of compound A that permeated skin | 59% | 64% | 59% | 63% | 66% |
| Evaluation 1: Amount of compound A that permeated skin | B | B | B | B | B |
| Evaluation 3: ratio of compound A that permeated skin | B | B | B | B | B |

Addition of a permeation enhancer to an ethanol solution having a maleate of compound A dissolved therein improved the skin permeation ratio from 23% to up to 66%.

**[Table 5]**

| | Comparative Example 5 | Example 22 | Example 23 | Example 24 | Example 25 |
|---|---|---|---|---|---|
| Maleate of compound A | 10 | 10 | 10 | 10 | 10 |
| Dimethyl sulfoxide | 90 | 89 | 89 | 89 | 89 |
| Oleic acid | | 1 | | | |
| Decanol | | | 1 | | |
| Lauryl alcohol | | | | 1 | |
| Oleyl alcohol | | | | | 1 |
| Amount of compound A that permeated skin (µg/cm²) | **3040** | **7830** | **7840** | **8830** | **5540** |
| Ratio of compound A that permeated skin | 30% | 78% | 78% | 88% | 55% |
| Evaluation 1: Amount of compound A that permeated skin | A | A | A | A | A |
| Evaluation 3: ratio of compound A that permeated skin | D | A | A | A | B |

Addition of a permeation enhancer to a dimethyl sulfoxide solution having a maleate of compound A dissolved therein improved the skin permeation ratio from 30% to up to 88%.

**[Table 6]**

| | Comparative Example 6 | Example 26 | Comparative Example 7 | Example 27 |
|---|---|---|---|---|
| Maleate of compound A | 10 | 10 | 10 | 10 |
| N-methyl pyrrolidone | 90 | 89 | | |
| Polyethylene glycol 200 | | | 90 | 89 |
| Lauryl alcohol | | 1 | | 1 |
| Amount of compound A that permeated skin (µg/cm²) | **270** | **7740** | **10** | **470** |
| Increase ratio of amount permeating skin by permeation enhancer | - | 29 | - | 47 |
| Evaluation 1: Amount of compound A that permeated skin | D | A | D | C |
| Evaluation 2: increase ratio of amount permeating skin due to permeation enhancer | - | A | - | A |

Addition of a permeation enhancer to an N-methyl pyrrolidone or polyethylene glycol solution having a maleate of compound A dissolved therein increased the amount permeating the skin by 29 times and 47 times, respectively.

**[Table 7]**

| | Comparative Example 8 | Example 28 | Example 29 | Example 30 | Example 31 |
|---|---|---|---|---|---|
| Maleate of compound A | 2.0 | 2.0 | 2.0 | 2.0 | 2.0 |
| Ethanol | 68.6 | 67.9 | 67.9 | 67.9 | 67.9 |
| Water | 29.4 | 29.1 | 29.1 | 29.1 | 29.1 |
| Lauric acid | | 1.0 | | | |
| Isostearic acid | | | 1.0 | | |
| Lauryl alcohol | | | | 1.0 | |
| Isostearyl alcohol | | | | | 1.0 |
| Amount of compound A that permeated skin (µg/cm²) | **370** | **1480** | **1360** | **1700** | **1570** |
| Ratio of compound A that permeated skin | 19% | 74% | 68% | 85% | 79% |
| Evaluation 1: Amount of compound A that permeated skin | D | B | B | A | B |
| Evaluation 3: ratio of compound A that permeated skin | D | B | B | A | A |

Addition of a permeation enhancer to an ethanol/water mixture solution having a maleate of compound A dissolved therein improved the skin permeation ratio from 19% to up to 85%.

**[Table 8]**

| | Comparative Example 9 | Example 32 | Comparative Example 10 | Example 33 | Comparative Example 11 | Example 34 |
|---|---|---|---|---|---|---|
| Maleate of compound A | 10 | 10 | 10 | 10 | 10 | 10 |
| Propylene glycol | 81 | 80.1 | 63 | 62.3 | 45 | 44.5 |
| Water | 9 | 8.9 | 27 | 26.7 | 45 | 44.5 |
| Hexanol | | | | | | 1 |
| Decanol | | | | 1 | | |
| Lauryl alcohol | | 1 | | | | |
| Amount of compound A that permeated skin (µg/cm²) | **400** | **7950** | **50** | **8550** | **220** | **870** |
| Increase ratio of amount permeating skin by permeation enhancer | 1 | 20 | 1 | 171 | 1 | 4 |
| Evaluation 1: Amount of compound A that permeated skin | C | A | D | A | D | B |
| Evaluation 2: increase ratio of amount permeating skin due to permeation enhancer | - | A | - | A | - | C |

Addition of a permeation enhancer to a propylene glycol/water mixture solution having a maleate of compound A dissolved therein increased the amount permeated through the skin by up to 171 times. In addition, from the results shown in Tables 7 and 8, it was clarified that the effect of the permeation enhancer was observed even with coexistence of water and the amount permeated through the skin tended to increase by the coexistence of water.

### <Example 35 and Comparative Example 12>

A transdermal preparation having a form of an adhesive preparation was produced by the following method.

A solution prepared by dissolving a maleate of compound A in dimethyl sulfoxide, an acrylic adhesive solution(DURO-TAK 387-2510, manufactured by Henkel Corporation), and a permeation enhancer were stirred in a container at a ratio shown in Table 9, and an adhesive solution containing the maleate of compound A was obtained. The obtained adhesive solution was applied to a polyethylene terephthalate (PET) film (thickness: 50 µm) base material and dried for 10 minutes with blowing hot air at 70°C to remove the solvent and form a coated layer. Thus a transdermal preparation (tape) having a form of an adhesive preparation with a 25 mg adhesive layer containing the active ingredient on the base material was obtained.

**[Table 9]**

| | Comparative Example 12 | Example 35 |
|---|---|---|
| Maleate of compound A | 10 | 10 |
| Dimethyl sulfoxide | 40 | 40 |
| Lauryl alcohol | | 16 |
| Acrylic adhesive agent | 50 | 34 |
| Amount of compound A that permeated skin (µg/cm²) | **70** | **1400** |
| Increase ratio of amount permeating skin by permeation enhancer | 1 | 20 |
| Evaluation 1: Amount of compound A that permeated skin | D | B |
| Evaluation 2: increase ratio of amount permeating skin due to permeation enhancer | - | A |

The amount of compound A permeating the skin was measured and evaluated by the same manner as described above except that each preparation of Comparative Example 12 and Example 35 was brought into contact with rat skin instead of uniformly applicating 100 mg/cm² of the composition to be evaluated on rat skin. The results are shown in Table 9. As is clear from these results, use of the composition of the present invention provided a prominent effect on increasing the amount that permeated the skin of a permeation enhancer even in the form of a transdermal preparation having an adhesive layer.

### <Example 36>

A transdermal preparation having a form of a gel was produced by the following method.

An aqueous gel was obtained by dissolving 1 g of hydroxypropyl cellulose (HPC-VH, Nippon Soda Co., Ltd.) in 8.8 g of water. A maleate of compound A, propylene glycol, water and decanol were stirred in a container at a ratio of 10:61.6:17.6:1 to prepare a solution of maleate of compound A. The obtained solution of maleate of compound A and the obtained aqueous gel were mixed at a ratio of 90.2:9.8, and agitated with a planetary centrifugal mixer (Awatori Rentaro ARE-310, Thinky Corporation) until the the mixture became uniform visually. Thus the composition for transdermal absorption was obtained. The planetary centrifugal mixer used in this examples was a general type of stirrer, which does not directly shear contents with a stirring blade.

### <Example 37>

A transdermal preparation having a form of a gel was produced by the following method.

An aqueous gel was obtained by dissolving 1 g of hydroxypropyl cellulose (HPC-VH, Nippon Soda Co., Ltd.) in 8.8 g of water. A maleate of compound A, propylene glycol, water and decanol were stirred in a container at a ratio of 10:60.9:17.4:2 to prepare a solution of maleate of compound A. The obtained solution of maleate of compound A and the obtained aqueous gel were mixed at a ratio of 90.3:9.7, and agitated with a planetary centrifugal mixer (Awatori Rentaro ARE-310, Thinky Corporation) until the mixture became unifrom visually. Thus the composition for transdermal absorption was obtained.

### <Example 38>

A transdermal preparation having a form of a gel was produced by the following method.

A solution of maleate of compound A was prepared with a maleate of compound A, propylene glycol, water and decanol at a ratio described in Table 10. To this solution with stirring in a container, polyvinyl pyrrolidone (Kollidon 90F, BASF) powder was added at a ratio described in Table 10. Thus a composition for transdermal absorption was obtained.

### <Example 39>

A transdermal preparation having a form of a gel was produced by the following method.

A solution of a mixture of a maleate of compound A, propylene glycol and decanol at a ratio of 10:58.1:2 was added to a gelatin aqueous solution dissolved by heating, and stirred in a container, so that a composition for transdermal absorption was obtained.

### <Example 40>

A transdermal preparation having a form of a gel was produced by the following method.

Polyvinyl pyrrolidone powder was added to a gelatin aqueous solution dissolved by heating. A solution of a mixture of a maleate of compound A, propylene glycol and decanol at a ratio of 10:55.3:2 was added to the aqueous solution, and stirred in a container, so that a composition for transdermal absorption was obtained.

### <Example 41>

A transdermal preparation having a form of a gel was produced by the following method.

A composition for transdermal absorption was obtained by the same method as in Example 39 except that gelatin in Example 39 was changed to agar.

### <Example 42>

A transdermal preparation having a form of a gel was produced by the following method.

A composition for transdermal absorption was obtained by the same method as in Example 40 except that gelatin in Example 40 was changed to agar.

### <Comparative Example 13>

A transdermal preparation having a form of a gel was produced by the following method.

A solution of maleate of compound A was prepared with a maleate of compound A, propylene glycol and water at a ratio of 1:29.0:29.0. While the obtained solution was stirred in a container, powder of a partially neutralized product of sodium polyacrylate (Aronvis AH, Toagosei Co., Ltd.) as a gelling agent was added at a ratio described in Table 11, so that a thickening fluid was obtained. Further, this thickening fluid was mixed with propylene glycol at a ratio of 61.5:38.6, so that a composition for transdermal absorption was obtained.

### <Example 43>

A transdermal preparation having a form of a gel was produced by the following method.

A solution of maleate of compound A was prepared with a maleate of compound A, propylene glycol and water at a ratio of 1:28.7:28.7. While the obtained solution was stirred in a container, powder of a partially neutralized product of sodium polyacrylate (Aronvis AH, Toagosei Co., Ltd.) as a gelling agent was added at a ratio described in Table 11, so that a thickening fluid was obtained. Further, this thickening fluid, propylene glycol and decanol were mixed at a ratio of 60.9:38.2:1, so that a composition for transdermal absorption was obtained.

### <Example 44>

A transdermal preparation having a form of a gel was produced by the following method.

A solution of maleate of compound A was prepared with a maleate of compound A, propylene glycol and water at a ratio of 1:28.4:28.4. While the obtained solution was stirred in a container, powder of a partially neutralized product of sodium polyacrylate (Aronvis AH, Toagosei Co., Ltd.) as a gelling agent was added at a ratio described in Table 11, so that a thickening fluid was obtained. Further, this thickening fluid, propylene glycol and decanol were mixed at a ratio of 60.3:37.8:2, so that a composition for transdermal absorption was obtained.

### <Example 45>

A composition for transdermal absorption of Example 41 was obtained in exactly the same manner as for Example 39 except that lauryl alcohol was used instead of decanol.

In the same manner as for Example 1, the amount of compound A permeating the skin was measured and evaluated by the same method as described above except for uniform application of preparations obtained in Examples 36 to 41 and Comparative Example 13; and results are shown in Tables 10 and 11.

**[Table 10-1]**

| | Example 33 | Example 36 | Example 37 | Example 38 |
|---|---|---|---|---|
| Maleate of compound A | 10.0 | 10.0 | 10.0 | 10.0 |
| Propylene glycol | 62.3 | 61.6 | 60.9 | 57.4 |
| Water | 26.7 | 26.4 | 26.1 | 24.6 |
| Decanol | 1.0 | 1.0 | 2.0 | 2.0 |
| Hydroxypropyl cellulose | | 1.0 | 1.0 | |
| Polyvinyl pyrrolidone | | | | 6.0 |
| Amount of compound A that permeated skin (µg/cm²) | **8550** | **7010** | **8410** | **7820** |
| Ratio of compound A that permeated skin | 86% | 70% | 84% | 78% |
| Evaluation 1: Amount of compound A that permeated skin | A | A | A | A |
| Evaluation 3: ratio of compound A that permeated skin | A | B | A | A |

**[Table 10-2]**

| | Example 39 | Example 40 | Example 41 | Example 42 |
|---|---|---|---|---|
| Maleate of compound A | 10.0 | 10.0 | 10.0 | 10.0 |
| Propylene glycol | 58.1 | 55.3 | 60.9 | 58.1 |
| Water | 24.9 | 23.7 | 26.1 | 24.9 |
| Decanol | 2.0 | 2.0 | 2.0 | 2.0 |
| Polyvinyl pyrrolidone | | 4.0 | | 4.0 |
| Gelatin | 5.0 | 5.0 | | |
| Agar | | | 1.0 | 1.0 |
| Amount of compound A that permeated skin (µg/cm²) | **8410** | **7820** | **8410** | **7820** |
| Ratio of compound A that permeated skin | 84% | 78% | 84% | 78% |
| Evaluation 1: Amount of compound A that permeated skin | A | A | A | A |
| Evaluation 3: ratio of compound A that permeated skin | A | A | A | A |

**[Table 11]**

| | Comparative Example 13 | Example 43 | Example 44 | Example 45 |
|---|---|---|---|---|
| Maleate of compound A | 1.0 | 1.0 | 1.0 | 1.0 |
| Propylene glycol | 67.6 | 66.9 | 66.2 | 66.9 |
| Water | 29.0 | 28.7 | 28.4 | 28.7 |
| Decanol | | 1.0 | 2.0 | |
| Lauryl alcohol | | | | 1.0 |
| Partially neutralized product of sodium polyacrylate | 2.5 | 2.5 | 2.5 | 2.5 |
| Amount of compound A that permeated skin (µg/cm²) | **30** | **510** | **730** | **430** |
| Increase ratio of amount permeating skin by permeation enhancer | 1 | 17 | 24 | 14 |
| Evaluation 1: Amount of compound A that permeated skin | D | C | B | C |
| Evaluation 2: increase ratio of amount permeating skin due to permeation enhancer | - | B | A | B |

As is clear from the results, an increasing effect of an permeation enhancer on the amount permeating the skin was prominent even when the composition of the present invention is in the form of a gel.

## Claims

1. A composition for external use comprising 1-(3-(2-(1-benzothiophen-5-yl)ethoxy)propyl)azetidin-3-ol or a salt thereof, one or more solvents selected from the group consisting of alcohols, sulfoxides and amides, and a permeation enhancer.

2. The composition for external use according to Claim 1, wherein the solvent is selected from the group consisting of ethanol, benzyl alcohol, propylene glycol, butylene glycol, dipropylene glycol, polyethylene glycol, dimethyl sulfoxide and N-methyl-2-pyrrolidone.

3. The composition for external use according to claim 1 or 2, further comprising water.

4. The composition for external use according to any one of claims 1 to 3, wherein the permeation enhancer is a compound having a carboxyl group, a hydroxyl group or an alkoxycarbonyl group.

5. The composition for external use according to any one of claims 1 to 4, wherein the permeation enhancer is a compound having an aliphatic group having 5 to 18 carbon atoms.

6. The composition for external use according to any one of claims 1 to 5, further comprising a water-soluble polymer.

7. The composition for external use according to claim 6, wherein the water-soluble polymer is selected from the group consisting of carrageenan, propylene glycol alginate, agar, gelatin, hydroxypropyl cellulose, polyvinyl pyrrolidone, carboxyvinyl polymer, sodium polyacrylate and polyvinyl alcohol.

8. The composition for external use according to claim 6, wherein the water-soluble polymer is selected from the group consisting of propylene glycol alginate, agar, gelatin, hydroxypropyl cellulose and polyvinyl pyrrolidone.

9. The composition for external use according to any one of claims 1 to 5, further comprising an adhesive agent.

10. The composition for external use according to any one of claims 1 to 9, wherein the composition is a transdermal composition for external use.

11. An adhesive preparation comprising the composition for external use according to any one of claims 1 to 10.

12. The adhesive preparation according to claim 11, wherein the adhesive preparation is a cataplasm or a patch.

13. The composition for external use according to any one of claims 1 to 8, wherein the composition is a transnasal composition for external use.
